(19) Europäisches Patentamt
European Patent Office
Office européen des brevets

(11) EP 2 272 847 A1

(12) EUROPEAN PATENT APPLICATION

(43) Date of publication:
**12.01.2011 Bulletin 2011/02**

(51) Int Cl.:
***C07D 451/02** (2006.01)* ***A61K 31/46** (2006.01)*
***A61P 25/00** (2006.01)*

(21) Application number: **10180734.5**

(22) Date of filing: **06.04.2006**

(84) Designated Contracting States:
**AT BE BG CH CY CZ DE DK EE ES FI FR GB GR HU IE IS IT LI LT LU LV MC NL PL PT RO SE SI SK TR**

(30) Priority: **08.04.2005 DK 200500505**
**11.04.2005 US 669918 P**
**11.11.2005 DK 200501572**
**15.11.2005 US 736331 P**

(62) Document number(s) of the earlier application(s) in accordance with Art. 76 EPC:
**06743248.4 / 1 869 033**

(71) Applicant: **NeuroSearch A/S**
**2750 Ballerup (DK)**

(72) Inventors:
- **Peters, Dan**
**2750, Ballerup (DK)**
- **Dahl, Bjarne, H.**
**3540, Lynge (DK)**
- **Olesen, Dorthe, Filtenborg**
**2750, Ballerup (DK)**
- **Nielsen, Elsebet, Østergaard**
**2750, Ballerup (DK)**
- **Olsen, Gunnar, M.**
**2765, Smørum (DK)**
- **Redrobe, John, Paul**
**2610, Rødovre (DK)**

(74) Representative: **Madsen, Inger Margrethe Schelde**
**NeuroSearch A/S**
**Patent Department**
**93 Pederstrupvej**
**2750 Ballerup (DK)**

Remarks:
This application was filed on 28-09-2010 as a divisional application to the application mentioned under INID code 62.

# (54) Enantiomers and their use as monoamine neurotransmitter re-uptake inhibitors

(57) This invention relates to novel enantiomers of Formula I useful as monoamine neurotransmitter re-uptake inhibitors.
In other aspects the invention relates to the use of these compounds in a method for therapy and to pharmaceutical compositions comprising the compounds of the invention.

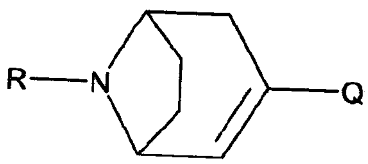

(I)

EP 2 272 847 A1

## Description

### TECHNICAL FIELD

This invention relates to novel enantiomers useful as monoamine neurotransmitter re-uptake inhibitors.

In other aspects the invention relates to the use of these compounds in a method for therapy and to pharmaceutical compositions comprising the compounds of the invention.

### BACKGROUND ART

WO 97/13770 (NeuroSearch A/S) describes a group of 8-aza-bicyclo[3.2.1]oct-2-ene derivatives. One of the compounds disclosed is the racemate (±)-3-(4-chlorophenyl)-8-H-8-aza-bicyclo[3.2.1]oct-2-ene (first compound of Example 3, page 21).

We have now discovered that the enantiomers of (±)-8-aza-bicyclo[3.2.1]oct-2-ene derivatives show an interesting pharmacological profile as monoamine reuptake inhibitors, in particular as regards the level of activity on reuptake of the monoamine neurotransmitters serotonin, dopamine and noradrenaline, such as the ratio of the serotonin reuptake versus the noradrenaline and dopamine reuptake activity.

Further it is often desirable, and sometimes subject to regulatory demands, to undertake drug development on specific enantiomers rather than racemic drugs. This rationale is based on the findings that often the desired characteristics of chiral compounds reside with one of its enantiomers, while the other enantiomer might in fact add to a potential toxicological effect of the drug.

Also, in order to allow thorough investigation of each enantiomer, enantiopure compounds and processes for obtaining enantiopure such compounds of chiral compounds are of significant importance for drug development.

### SUMMARY OF THE INVENTION

In its first aspect, the invention provides an enantiopure compound of Formula I

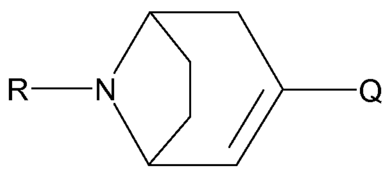

(I)

or a pharmaceutically acceptable salt thereof; wherein R and Q are as defined below.

In its second aspect, the invention provides an enantiopure compound of 3-(4-chlorophenyl)-8-H-8-aza-bicyclo [3.2.1]oct-2-ene; or a pharmaceutically acceptable salt thereof.

In its third aspect, the invention provides a pharmaceutical composition, comprising a therapeutically effective amount of a compound of the invention, or a pharmaceutically acceptable salt thereof, together with at least one pharmaceutically acceptable carrier, excipient or diluent.

In a further aspect, the invention provides the use of a compound of the invention, or a pharmaceutically acceptable salt thereof, for the manufacture of a pharmaceutical composition for the treatment, prevention or alleviation of a disease or a disorder or a condition of a mammal, including a human, which disease, disorder or condition is responsive to inhibition of monoamine neurotransmitter re-uptake in the central nervous system.

In a still further aspect, the invention relates to a method for treatment, prevention or alleviation of a disease or a disorder or a condition of a living animal body, including a human, which disorder, disease or condition is responsive to responsive to inhibition of monoamine neurotransmitter re-uptake in the central nervous system, which method comprises the step of administering to such a living animal body in need thereof a therapeutically effective amount of a compound of the invention, or a pharmaceutically acceptable salt thereof.

Other objects of the invention will be apparent to the person skilled in the art from the following detailed description and examples.

## DETAILED DISCLOSURE OF THE INVENTION

### Enantiopure compounds

In its first aspect, the invention provides an enantiopure compound of Formula I

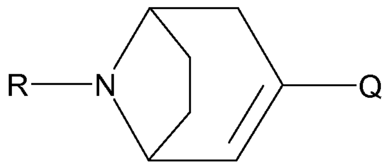

(I)

or a pharmaceutically acceptable salt thereof;
wherein
R represents hydrogen or alkyl;
which alkyl is optionally substituted with one or more substituents independently selected from the group consisting of:

halo, trifluoromethyl, trifluoromethoxy, cyano, hydroxy, amino, nitro, alkoxy, cycloalkoxy, alkyl, cycloalkyl, cycloalkylalkyl, alkenyl and alkynyl; and

Q represents an aryl group;
which heteroaryl group is optionally substituted with one or more substituents independently selected from the group consisting of:

halo, trifluoromethyl, trifluoromethoxy, cyano, nitro, hydroxy, alkoxy, cycloalkoxy, alkoxyalkyl, cycloalkoxyalkyl, alkyl, cycloalkyl, cycloalkylalkyl, alkenyl, alkynyl, -NR'R", -(C=O)NR'R" or -NR'(C=O)R";
wherein R' and R" independent of each other are hydrogen or alkyl.

In one embodiment, R represents hydrogen or alkyl. In a special embodiment, R represents hydrogen. In a further embodiment, R represents alkyl, such as methyl.

In a second embodiment, Q represents phenyl, which phenyl group is optionally substituted with one or more substituents independently selected from the group consisting of: halo, trifluoromethyl, trifluoromethoxy, cyano, hydroxy and alkoxy. In a further embodiment, Q represents phenyl, which phenyl group is optionally substituted with one or more substituents independently selected from the group consisting of: halo, trifluoromethyl, trifluoromethoxy, cyano and alkoxy. In a still further embodiment, Q represents phenyl, which phenyl group is optionally substituted with one or two halo. In a special embodiment, Q represents halophenyl, such as chlorophenyl. In particular, Q represents 2-chlorophenyl, 3-chlorophenyl or 4-chlorophenyl. In a further embodiment, Q represents dihalophenyl, such as dichlorophenyl. In particular, Q represents 2,3-dichlorophenyl or 3,4-dichlorophenyl.

In a further embodiment, Q represents naphthyl, which naphthyl group is optionally substituted with one or more substituents independently selected from the group consisting of: halo, trifluoromethyl, trifluoromethoxy, cyano, hydroxy and alkoxy. In a still further embodiment, Q represents naphthyl, which naphthyl group is optionally substituted with one or more substituents independently selected from the group consisting of: halo, trifluoromethyl, trifluoromethoxy, cyano and alkoxy. In a further embodiment, Q represents naphthyl, which naphthyl group is optionally substituted with hydroxy or alkoxy. In a special embodiment, Q represents naphthyl, such as naphthalen-2-yl. In a still further embodiment, Q represents alkoxynaphthyl, such as methoxynaphthyl, such as 6-methoxy-naphthalen-2-yl. In a further embodiment, Q represents hydroxynaphthyl, such as 6-hydroxy-naphthalen-2-yl.

In a further embodiment, the present invention provides an enantiopure compound of

3-(4-Chlorophenyl)-8-H-8-aza-bicyclo[3.2.1]oct-2-ene;
3-Naphthalen-2-yl-8-aza-bicyclo[3.2.1]oct-2-ene;
3-(2-Chlorophenyl)-8-H-8-azabicyclo[3.2.1]oct-2-ene;
3-(2,3-Dichlorophenyl)-8-H-8-azabicyclo[3.2.1]oct-2-ene;
3-(3-Chlorophenyl)-8-H-8-azabicyclo[3.2.1]oct-2-ene;
3-(3,4-Dichlorophenyl) 8-H-8-azabicyclo[3.2.1]oct-2-ene;
3-(4-Chlorophenyl)-8-methyl-8-aza-bicyclo[3.2.1]oct-2-ene;
3-(2-Chlorophenyl)-8-methyl-8-azabicyclo[3.2.1]oct-2-ene;

3-(2,3-Dichlorophenyl)-8-methyl-8-azabicyclo[3.2.1]oct-2-ene;
3-(3,4-Dichlorophenyl)-8-methyl-8-azabicyclo[3.2.1]oct-2-ene;
3-(3-Chlorophenyl)-8-methyl-8-azabicyclo[3.2.1]oct-2-ene;
3-(6-Methoxy-naphthalen-2-yl)-8-aza-bicyclo[3.2.1]oct-2-ene;
8-Methyl-3-(naphthalen-2-yl)-8-aza-bicyclo[3.2.1]oct-2-ene
3-(6-Methoxy-naphthalen-2-yl)-8-methyl-8-aza-bicyclo[3.2.1]oct-2-ene;
6-(8-Aza-bicyclo[3.2.1]oct-2-en-3-yl)-naphthalen-2-ol;
or a pharmaceutically acceptable salt thereof.

In a still further embodiment, the chemical compound of the invention is enantiopure (+)-3-(4-Chlorophenyl)-8-H-8-aza-bicyclo[3.2.1]oct-2-ene; or a pharmaceutically acceptable salt thereof.

In a further embodiment, the chemical compound of the invention is enantiopure (-)-3-(4-Chlorophenyl)-8-H-8-aza-bicyclo[3.2.1]oct-2-ene; or a pharmaceutically acceptable salt thereof.

In a still further embodiment, the chemical compound of the invention is enantiopure (+)-3-(2-Chlorophenyl)-8-H-8-azabicyclo[3.2.1]oct-2-ene; enantiopure (+)-3-(2,3-Dichlorophenyl)-8-H-8-azabicyclo[3.2.1]oct-2-ene; enantiopure (+)-3-(3-Chlorophenyl)-8-H-8-azabicyclo[3.2.1]oct-2-ene; enantiopure (+)-3-(3,4-Dichlorophenyl) 8-H-8-azabicyclo [3.2.1]oct-2-ene; enantiopure (-)-3-(2-Chlorophenyl)-8-H-8-azabicyclo[3.2.1]oct-2-ene; enantiopure (-)-3-(2,3-Dichlorophenyl)-8-H-8-azabicyclo[3.2.1]oct-2-ene; enantiopure (-)-3-(3-Chlorophenyl)-8-H-8-azabicyclo[3.2.1]oct-2-ene; enantiopure (-)-3-(3,4-Dichlorophenyl)-8-H-8-azabicyclo[3.2.1]oct-2-ene; enantiopure (-)-3-(4-Chlorophenyl)-8-methyl-8-aza-bicyclo[3.2.1]oct-2-ene; enantiopure (-)-3-(2-Chlorophenyl)-8-methyl-8-azabicyclo[3.2.1]oct-2-ene; enantiopure (-)-3-(2,3-Dichlorophenyl)-8-methyl-8-azabicyclo[3.2.1]oct-2-ene; enantiopure (-)-3-(3,4-Dichlorophenyl)-8-methyl-8-azabicyclo[3.2.1]oct-2-ene; enantiopure (+)-3-(4-Chlorophenyl)-8-methyl-8-aza-bicyclo[3.2.1]oct-2-ene; enantiopure (+)-3-(2-Chlorophenyl)-8-methyl-8-azabicyclo[3.2.1]oct-2-ene; enantiopure (+)-3-(2,3-Dichlorophenyl)-8-methyl-8-azabicyclo[3.2.1]oct-2-ene; enantiopure (+)-3-(3-Chlorophenyl)-8-methyl-8-azabicyclo[3.2.1]oct-2-ene; enantiopure (+)-3-(3,4-Dichlorophenyl)-8-methyl-8-azabicyclo[3.2.1]oct-2-ene; enantiopure (+)-3-Naphthalen-2-yl-8-aza-bicyclo[3.2.1]oct-2-ene; enantiopure (+)-3-(6-Methoxy-naphthalen-2-yl)-8-aza-bicyclo[3.2.1]oct-2-ene; enantiopure (-)-3-Naphthalen-2-yl-8-aza-bicyclo[3.2.1]oct-2-ene; enantiopure (-)-3-(6-Methoxy-naphthalen-2-yl)-8-aza-bicyclo[3.2.1]oct-2-ene; enantiopure (-)-8-Methyl-3-(naphthalen-2-yl)-8-aza-bicyclo[3.2.1]oct-2-ene; enantiopure (-)-3-(6-Methoxy-naphthalen-2-yl)-8-methyl-8-aza-bicyclo[3.2.1]oct-2-ene; enantiopure (+)-8-Methyl-3-(naphthalen-2-yl)-8-aza-bicyclo[3.2.1]oct-2-ene; enantiopure (+)-3-(6-Methoxy-naphthalen-2-yl)-8-methyl-8-aza-bicyclo[3.2.1]oct-2-ene;
enantiopure (+)-6-(8-Aza-bicyclo[3.2.1]oct-2-en-3-yl)-naphthalen-2-ol;
or a pharmaceutically acceptable salt thereof.

In a further aspect, the invention provides a pharmaceutical composition, comprising a therapeutically effective amount of (±)-3-naphthalen-2-yl-8-aza-bicyclo[3.2.1]oct-2-eneany of its isomers or any mixture of its isomers, or a pharmaceutically acceptable salt thereof, together with at least one pharmaceutically acceptable carrier, excipient or diluent. Also, the invention provides the use of said pharmaceutical composition, for the manufacture of a pharmaceutical composition for the treatment, prevention or alleviation of a disease or a disorder or a condition of a mammal, including a human, which disease, disorder or condition is responsive to inhibition of monoamine neurotransmitter re-uptake in the central nervous system. Also, the invention provides a method for treatment, prevention or alleviation of a disease or a disorder or a condition of a living animal body, including a human, which disorder, disease or condition is responsive to responsive to inhibition of monoamine neurotransmitter re-uptake in the central nervous system, which method comprises the step of administering to such a living animal body in need thereof a therapeutically effective amount of said pharmaceutical composition.

Any combination of two or more of the embodiments as described above is considered within the scope of the present invention.

Definition of Substituents

In the context of this invention halo represents fluoro, chloro, bromo or iodo.

In the context of this invention an alkyl group designates a univalent saturated, straight or branched hydrocarbon chain. The hydrocarbon chain preferably contains of from one to six carbon atoms ($C_{1-6}$-alkyl), including pentyl, isopentyl, neopentyl, tertiary pentyl, hexyl and isohexyl. In a preferred embodiment alkyl represents a $C_{1-4}$-alkyl group, including butyl, isobutyl, secondary butyl, and tertiary butyl. In another preferred embodiment of this invention alkyl represents a $C_{1-3}$-alkyl group, which may in particular be methyl, ethyl, propyl or isopropyl.

In the context of this invention an alkenyl group designates a carbon chain containing one or more double bonds, including di-enes, tri-enes and poly-enes. In a preferred embodiment the alkenyl group of the invention comprises of from two to six carbon atoms ($C_{2-6}$-alkenyl), including at least one double bond. In a most preferred embodiment the alkenyl group of the invention is ethenyl; 1- or 2-propenyl; 1-, 2- or 3-butenyl, or 1,3-butadienyl; 1-, 2-, 3-, 4- or 5-hexenyl,

or 1,3-hexadienyl, or 1,3,5-hexatrienyl.

In the context of this invention an alkynyl group designates a carbon chain containing one or more triple bonds, including di-ynes, tri-ynes and poly-ynes. In a preferred embodiment the alkynyl group of the invention comprises of from two to six carbon atoms ($C_{2-6}$-alkynyl), including at least one triple bond. In its most preferred embodiment the alkynyl group of the invention is ethynyl; 1-, or 2-propynyl; 1-, 2-, or 3-butynyl, or 1,3-butadiynyl; 1-, 2-, 3-, 4-pentynyl, or 1,3-pentadiynyl; 1-, 2-, 3-, 4-, or 5-hexynyl, or 1,3-hexadiynyl or 1,3,5-hexatriynyl.

In the context of this invention a cycloalkyl group designates a cyclic alkyl group, preferably containing of from three to seven carbon atoms ($C_{3-7}$cycloalkyl), including cyclopropyl, cyclobutyl, cyclopentyl, cyclohexyl and cycloheptyl.

Alkoxy is O-alkyl, wherein alkyl is as defined above.

Cycloalkoxy means O-cycloalkyl, wherein cycloalkyl is as defined above.

Cycloalkylalkyl means cycloalkyl as above and alkyl as above, meaning for example, cyclopropylmethyl.

Amino is $NH_2$ or NH-alkyl or N-$(alkyl)_2$, wherein alkyl is as defined above.

In the context of this invention an aryl group designates a carbocyclic aromatic ring system such as phenyl, naphthyl (1-naphthyl or 2-naphthyl) or fluorenyl.

## Enantiopurity

In the context of this invention a compound being enantiopure means that the compound is in enantiomeric excess of at least 95.0% (w/w) over the opposite enantiomer. In one embodiment, the enantiopure compound is in enantiomeric excess of at least 97.0%, 98.0% or 99.0% over the opposite enantiomer. In a further embodiment, the enantiopure compound is in enantiomeric excess of at least 99.2%, 99.5%, or 99.7% over the opposite enantiomer. In a still further embodiment, the enantiopure compound is in enantiomeric excess of at least 99.90% or 99.95% over the opposite enantiomer.

## Pharmaceutically Acceptable Salts

The chemical compound of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate, the phthalate, the salicylate, the sorbate, the stearate, the succinate, the tartrate, the toluene-p-sulphonate, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.

Examples of pharmaceutically acceptable cationic salts of a chemical compound of the invention include, without limitation, the sodium, the potassium, the calcium, the magnesium, the zinc, the aluminium, the lithium, the choline, the lysinium, and the ammonium salt, and the like, of a chemical compound of the invention containing an anionic group. Such cationic salts may be formed by procedures well known and described in the art.

In the context of this invention the "onium salts" of N-containing compounds are also contemplated as pharmaceutically acceptable salts. Preferred "onium salts" include the alkyl-onium salts, the cycloalkyl-onium salts, and the cycloalkylalkyl-onium salts.

Examples of pre- or prodrug forms of the chemical compound of the invention include examples of suitable prodrugs of the substances according to the invention include compounds modified at one or more reactive or derivatizable groups of the parent compound. Of particular interest are compounds modified at a carboxyl group, a hydroxyl group, or an amino group. Examples of suitable derivatives are esters or amides.

The chemical compound of the invention may be provided in dissoluble or indissoluble forms together with a pharmaceutically acceptable solvent such as water, ethanol, and the like. Dissoluble forms may also include hydrated forms such as the monohydrate, the dihydrate, the hemihydrate, the trihydrate, the tetrahydrate, and the like. In general, the dissoluble forms are considered equivalent to indissoluble forms for the purposes of this invention.

## Labelled Compounds

The compounds of the invention may be used in their labelled or unlabelled form. In the context of this invention

the labelled compound has one or more atoms replaced by an atom having an atomic mass or mass number different from the atomic mass or mass number usually found in nature. The labelling will allow easy quantitative detection of said compound.

The labelled compounds of the invention may be useful as diagnostic tools, radio tracers, or monitoring agents in various diagnostic methods, and for *in vivo* receptor imaging.

The labelled isomer of the invention preferably contains at least one radionuclide as a label. Positron emitting radionuclides are all candidates for usage. In the context of this invention the radionuclide is preferably selected from $^{2}H$ (deuterium), $^{3}H$ (tritium), $^{13}C$, $^{14}C$, $^{131}I$, $^{125}I$, $^{123}I$, and $^{18}F$.

The physical method for detecting the labelled isomer of the present invention may be selected from Position Emission Tomography (PET), Single Photon Imaging Computed Tomography (SPECT), Magnetic Resonance Spectroscopy (MRS), Magnetic Resonance Imaging (MRI), and Computed Axial X-ray Tomography (CAT), or combinations thereof.

**Methods of Preparation**

The chemical compounds of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

**Biological Activity**

Compounds of the invention may be tested for their ability to inhibit reuptake of the monoamines dopamine, noradrenaline and serotonin in synaptosomes e.g. such as described in WO 97/30997 (NeuroSearch A/S). Based on the balanced activity observed in these tests the compound of the invention is considered useful for the treatment, prevention or alleviation of a disease or a disorder or a condition of a mammal, including a human, which disease, disorder or condition is responsive to inhibition of monoamine neurotransmitter re-uptake in the central nervous system.

In a special embodiment, the compounds of the invention are considered useful for the treatment, prevention or alleviation of: mood disorder, depression, atypical depression, depression secondary to pain, major depressive disorder, dysthymic disorder, bipolar disorder, bipolar I disorder, bipolar II disorder, cyclothymic disorder, mood disorder due to a general medical condition, substance-induced mood disorder, pseudodementia, Ganser's syndrome, obsessive compulsive disorder, panic disorder, panic disorder without agoraphobia, panic disorder with agoraphobia, agoraphobia without history of panic disorder, panic attack, memory deficits, memory loss, attention deficit hyperactivity disorder, obesity, anxiety, generalized anxiety disorder, eating disorder, Parkinson's disease, parkinsonism, dementia, dementia of ageing, senile dementia, Alzheimer's disease, acquired immunodeficiency syndrome dementia complex, memory dysfunction in ageing, specific phobia, social phobia, social anxiety disorder, post-traumatic stress disorder, acute stress disorder, drug addiction, drug abuse, cocaine abuse, nicotine abuse, tobacco abuse, alcohol addiction, alcoholism, kleptomania, pain, chronic pain, inflammatory pain, neuropathic pain, migraine pain, tension-type headache, chronic tension-type headache, pain associated with depression, fibromyalgia, arthritis, osteoarthritis, rheumatoid arthritis, back pain, cancer pain, irritable bowel pain, irritable bowel syndrome, post-operative pain, post-mastectomy pain syndrome (PMPS), post-stroke pain, drug-induced neuropathy, diabetic neuropathy, sympathetically-maintained pain, trigeminal neuralgia, dental pain, myofacial pain, phantom-limb pain, bulimia, premenstrual syndrome, premenstrual dysphoric disorder, late luteal phase syndrome, post-traumatic syndrome, chronic fatigue syndrome, urinary incontinence, stress incontinence, urge incontinence, nocturnal incontinence, sexual dysfunction, premature ejaculation, erectile difficulty, erectile dysfunction, premature female orgasm, restless leg syndrome, periodic limb movement disorder, eating disorders, anorexia nervosa, sleep disorders, pervasive developmental disorders, autism, Asperger's disorder, Rett's disorder, childhood disintegrative disorder, learning disabilities, motor skills disorders, mutism, trichotillomania, narcolepsy, post-stroke depression, stroke-induced brain damage, stroke-induced neuronal damage, Gilles de la Tourettes disease, tinnitus, tic disorders, body dysmorphic disorders, oppositional defiant disorder or post-stroke disabilities. In a preferred embodiment, the compounds are considered useful for the treatment, prevention or alleviation of depression.

It is at present contemplated that a suitable dosage of the active pharmaceutical ingredient (API) is within the range of from about 0.1 to about 1000 mg API per day, more preferred of from about 10 to about 500 mg API per day, most preferred of from about 30 to about 100 mg API per day, dependent, however, upon the exact mode of administration, the form in which it is administered, the indication considered, the subject and in particular the body weight of the subject involved, and further the preference and experience of the physician or veterinarian in charge.

**Pharmaceutical Compositions**

In another aspect the invention provides novel pharmaceutical compositions comprising a therapeutically effective amount of the chemical compound of the invention.

While a chemical compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the chemical compound of the invention, or a pharmaceutically acceptable salt or derivative thereof, together with one or more pharmaceutically acceptable carriers, and, optionally, other therapeutic and/or prophylactic ingredients, known and used in the art. The carrier(s) must be "acceptable" in the sense of being compatible with the other ingredients of the formulation and not harmful to the recipient thereof.

Pharmaceutical compositions of the invention may be those suitable for oral, rectal, bronchial, nasal, pulmonal, topical (including buccal and sub-lingual), transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

The chemical compound of the invention, together with a conventional adjuvant, carrier, or diluent, may thus be placed into the form of pharmaceutical compositions and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The chemical compound of the present invention can be administered in a wide variety of oral and parenteral dosage forms. It will be obvious to those skilled in the art that the following dosage forms may comprise, as the active component, either a chemical compound of the invention or a pharmaceutically acceptable salt of a chemical compound of the invention.

For preparing pharmaceutical compositions from a chemical compound of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid, which is in a mixture with the finely divided active component.

In tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify.

Compositions suitable for vaginal administration may be presented as pessaries, tampons, creams, gels, pastes, foams or sprays containing in addition to the active ingredient such carriers as are known in the art to be appropriate.

Liquid preparations include solutions, suspensions, and emulsions, for example, water or water-propylene glycol solutions. For example, parenteral injection liquid preparations can be formulated as solutions in aqueous polyethylene glycol solution.

The chemical compound according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may

take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations, intended for conversion shortly before use to liquid form preparations for oral administration. Such liquid forms include solutions, suspensions, and emulsions. In addition to the active component such preparations may comprise colorants, flavours, stabilisers, buffers, artificial and natural sweeteners, dispersants, thickeners, solubilizing agents, and the like.

For topical administration to the epidermis the chemical compound of the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co., Easton, PA).

A therapeutically effective dose refers to that amount of active ingredient, which ameliorates the symptoms or condition. Therapeutic efficacy and toxicity, e.g. $ED_{50}$ and $LD_{50}$, may be determined by standard pharmacological procedures in cell cultures or experimental animals. The dose ratio between therapeutic and toxic effects is the therapeutic index and may be expressed by the ratio $LD_{50}/ED_{50}$. Pharmaceutical compositions exhibiting large therapeutic indexes are preferred.

The dose administered must of course be carefully adjusted to the age, weight and condition of the individual being treated, as well as the route of administration, dosage form and regimen, and the result desired, and the exact dosage should of course be determined by the practitioner.

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of

from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 μg/kg i.v. and 1 μg/kg p.o. The upper limit of the dosage range is presently considered to be about 10 mg/kg i.v. and 100 mg/kg p.o. Preferred ranges are from about 0.1 μg/kg to about 10 mg/kg/day i.v., and from about 1 μg/kg to about 100 mg/kg/day p.o.

**Methods of Therapy**

In another aspect the invention provides a method for the treatment, prevention or alleviation of a disease or a disorder or a condition of a living animal body, including a human, which disease, disorder or condition is responsive to inhibition of monoamine neurotransmitter re-uptake in the central nervous system, and which method comprises administering to such a living animal body, including a human, in need thereof an effective amount of a chemical compound of the invention.

It is at present contemplated that suitable dosage ranges are 0.1 to 1000 milligrams daily, 10-500 milligrams daily, and especially 30-100 milligrams daily, dependent as usual upon the exact mode of administration, form in which administered, the indication toward which the administration is directed, the subject involved and the body weight of the subject involved, and further the preference and experience of the physician or veterinarian in charge.

## EXAMPLES

The invention is further illustrated with reference to the following examples, which are not intended to be in any way limiting to the scope of the invention as claimed.

**General:** All reactions involving air sensitive reagents or intermediates were performed under nitrogen and in anhydrous solvents. Magnesium sulphate was used as drying agent in the workup-procedures and solvents were evaporated under reduced pressure.

The chiral purities of the products can be analyzed by the following CE method: Capillary: Fused Silica, Bubble cell, L = 56 cm, l = 50 cm, 50 μm i.d. Temperature: 15°C. Voltage: 30 kV. Injection: 50 mbar in 2 sec. Detection: UV 255 nm. Running buffer: 25 mM Carboxymethylated-β-cyclodextrin in 50 mM phosphate buffer pH 7.0 containing 10% v/v acetonitrile.

### Example 1

#### Method A

**(+)-3-(4-Chlorophenyl)-8-H-8-aza-bicyclo[3.2.1]oct-2-ene D-tartaric acid salt**

A mixture of (-)-3-(4-chlorophenyl)-8-methyl-8-aza-bicyclo[3.2.1]oct-2-ene (5.3 g, 22.6 mmol), 2,2,2-trichloroethylchloroformate (9.3 ml, 67.8 mmol) and toluene (100 ml) was stirred at room temperature for 1 h. The mixture was stirred at 100°C for 15 h. Water (100 ml) was added and the mixture was stirred for 20 min at room temperature. The organic phase was separated and was washed with water (2 x 50 ml). The organic phase was evaporated. Zinc-dust (5.0 g, 76.5 mmol), acetic acid (50 ml) and water (25 ml) were added followed by stirring for 15 h. The mixture was made alkaline by adding ice (100 g) and concentrated ammonia (100 ml). The mixture was extracted with dichloromethane (2 x 50 ml) and was isolated as an oil. Ethanol (50 ml, 96%) and D-tartaric acid (3.0 g, 20.0 mmol) were added and was heated to reflux where the mixture became clear and was allowed to crystallize followed by filtration. The crystals were recrystallized a second time by the same procedure including purification of the clear mixture with active carbon (followed by filtration of the mixture). Yield 1.0 g (12%). Mp 178.8-179.6°C.

**(+)-3-(2-Chlorophenyl)-8-H-8-azabicyclo[3.2.1]oct-2-ene D-tartaric acid salt**

Was prepared according to method A. Mp 206.8-217.2°C.

**(+)-3-(2,3-Dichlorophenyl)-8-H-8-azabicyclo[3.2.1]oct-2-ene D-tartaric acid salt**

Was prepared according to method A. Mp 213.9°C.

**(+)-3-(3-Chlorophenyl)-8-H-8-azabicyclo[3.2.1]oct-2-ene D-tartaric acid salt**

Was prepared according to method A. Mp 178.4-183.0°C.

**(+)-3-(3,4-Dichlorophenyl) 8-H-8-azabicyclo[3.2.1]oct-2-ene D-tartaric acid salt**

Was prepared according to method A. Mp 162-184°C.

**(-)-3-(4-Chlorophenyl)-8-H-8-aza-bicyclo[3.2.1]oct-2-ene L-tartaric acid salt**

Was prepared according to method A from (+)-3-(4-chlorophenyl)-8-methyl-8-aza-bicyclo[3.2.1]oct-2-ene and L-tartaric acid Yield 35%. Mp 177.1-180.0°C.

**(-)-3-(2-Chlorophenyl)-8-H-8-azabicyclo[3.2.1]oct-2-ene L-tartaric acid salt**

Was prepared according to method A. Mp 204.6-206.8°C.

**(-)-3-(2,3-Dichlorophenyl)-8-H-8-azabicyclo[3.2.1]oct-2-ene L-tartaric acid salt**

Was prepared according to method A. Mp 210.4-211.4°C.

**(-)-3-(3-Chlorophenyl)-8-H-8-azabicyclo[3.2.1]oct-2-ene L-tartaric acid salt**

Was prepared according to method A. Mp 175.8-177.4°C.

**(-)-3-(3,4-Dichlorophenyl)-8-H-8-azabicyclo[3.2.1]oct-2-ene L-tartaric acid salt**

Was prepared according to method A. Mp 174.4-177.5°C.

**Method B**

**(-)-3-(4-Chlorophenyl)-8-methyl-8-aza-bicyclo[3.2.1]oct-2-ene hydrochloric acid salt**

A mixture of (-)-8-methyl-3-(trifluoromethylsulfonyloxy)-8-aza-bicyclo[3.2.1]oct-2-ene, (10.4 g 38 mmol), 1,2-dimethoxyetane (150 ml), 4-chlorophenylboronic acid (7.35 g, 47 mmol), potassium carbonate (20 g, 145 mmol), lithium chloride (2.0 g, 47 mmol) and water (75 ml) was bubbled through with argon for 10 min. Pd(PPh3)4 (0.17 g, 0.15 mmol) was added followed by reflux for 1 h. The mixture was allowed to cool to room temperature. Water (100 ml) was added followed by extraction with diethyl ether (2 x 50 ml). The organic phase was washed with water (2 x 50 ml). The organic phase was extracted with hydrochloric acid (30 ml, 4 M) and with water (2 x 30 ml). The acidic aqueous phase was made alkaline by adding concentrated aqueous ammonia (50 ml). The free base was precipitated and isolated by filtration. . $[\alpha]_D^{25}$ = (-)-30.8°. Yield 6.82 g (77%). The free base was converted to the hydrochloric acid salt. Mp 193.7°C.

**(-)-3-(2-Chlorophenyl)-8-methyl-8-azabicyclo[3.2.1]oct-2-ene hydrochloric acid salt**

Was prepared according to method B. Mp 274.8-275.3°C.

**(-)-3-(2,3-Dichlorophenyl)-8-methyl-8-azabicyclo[3.2.1]oct-2-ene hydrochloric acid salt**

Was prepared according to method B. Mp 225.9-231.5°C.

**(-)-3-(3,4-Dichlorophenyl)-8-methyl-8-azabicyclo[3.2.1]oct-2-ene hydrochloric acid salt**

Was prepared according to method B. Mp 228.5°C.

**(+)-3-(4-Chlorophenyl)-8-methyl-8-aza-bicyclo[3.2.1]oct-2-ene L-tartaric acid salt**

Was prepared according to method B from (+)-8-methyl-3-(trifluoromethylsulfonyloxy)-8-aza-bicyclo[3.2.1]oct-2-ene. As L-tartaric salt: $[\alpha]_D^{25}$ = (+)-19.2°, as free base $[\alpha]_D^{25}$ = (+)-37.4°. Recrystallized as L-tartaric salt, , $[\alpha]_D^{25}$ = (+)-20.3°, re-liberated as free base $[\alpha]_D^{25}$ = (+)-43.9°. Mp 135-160°C.

**(+)-3-(2-Chlorophenyl)-8-methyl-8-azabicyclo[3.2.1]oct-2-ene hydrochloric acid salt**

Was prepared according to method B. Mp 268.8°C.

**(+)-3-(2,3-Dichlorophenyl)-8-methyl-8-azabicyclo[3.2.1]oct-2-ene hydrochloric acid salt**

Was prepared according to method B. Mp 238°C.

**(+)-3-(3-Chlorophenyl)-8-methyl-8-azabicyclo[3.2.1]oct-2-ene hydrochloric acid salt**

Was prepared according to method B. Mp 102.3-122.3°C.

**(+)-3-(3,4-Dichlorophenyl)-8-methyl-8-azabicyclo[3.2.1]oct-2-ene hydrochloric acid salt**

Was prepared according to method B. Mp 242°C.

**Method C**

**(-)-8-Methyl-3-(trifluoromethylsulfonyloxy)-8-aza-bicyclo[3.2.1]oct-2-ene**

To a stirred mixture of [S-(R*, R*)](-)-bis-α-methyl-benzylamine hydrochloric acid salt $[\alpha]_D^{25}$ = (-)-73.2°) (86.5 g, 0.33 mmol) and tetrahydrofuran (1000 ml) was added at <5°C: Butyllithium (264 ml, 2.5 M). The mixture was stirred at 0°C for 1 h. The mixture was cooled to -70°C and tropinone (41.8 g, 0.3 mmol) solved in tetrahydrofuran (200 ml) was added over a period of 90 min. The mixture was stirred for 3 h at -70°C. N-phenyl-bis(trifluoromethanesulfon) imide (114.3 g, 0.32 mmol) solved in tetrahydrofuran was added to the mixture <70°C over 2 h time period. The mixture was allowed to reach room temperature over night. Water (3L) was added followed by extraction with diethylether (2 x 1 L). The organic phase was washed with water (2 x 1 L). The crude mixture of the title product and the chiral amine was separated by silica gel (1 kg) column chromatography using ethyl acetate initially in order to eluate the chiral amine and then use a mixture of methanol and dichloromethane (2 : 8) for the chiral triflate. The chiral triflate was isolated in 78% (0.233 mol).

**(+)-8-Methyl-3-(trifluoromethylsulfonyloxy)-8-aza-bicyclo[3.2.1]oct-2-ene**

Was prepared according to method C using the other chiral amine [R-(R*, R*)](+)-bis-α-methyl-benzylamine hydrochloric acid salt, , $[\alpha]_D^{25}$ = (+)-73.8°.

**(±)-8-Methyl-3-naphthalen-2-yl-8-aza-bicyclo[3.2.1]oct-2-ene hydrochloric acid salt**

Butyllithium in hexanes (21.2 ml, 53.0 mmol) was added dropwise to a solution of 2-bromonaphthalene (10 g,

48 mmol) in diethylether (100 ml) at -70°C. The mixture was stirred for 30 min. Tropinone (6.1 g, 44 mmol), solved in THF (50 ml) was added to the mixture at -70°C. The mixture was stirred for 2 h at -70°C and was then allowed to warm up to room temperature. The mixture was made acidic by adding aqueous hydrochloric acid (200 ml, 1 M). The mixture was washed with diethylether (2 x 100 ml) in order to remove impurities. The mixture was made alkaline by adding aqueous sodium hydroxide (250 ml, 1 M). The aqueous phase was extracted with diethyl ether. The organic phase was dried and evaporated. A mixture of the intermediate alcohol, acetic acid (50 ml) and concentrated hydrochloric acid (25 ml) was stirred at reflux for 1 h. The mixture was evaporated and made alkaline by adding concentrated aqueous ammonia (100 ml) followed by extraction with diethylether. The organic phase was dried and evaporated. Yield (free base) 3.8 g (32%). Mp (hydrochloride) 264-269°C

**(±)-3-Naphthalen-2-yl-8-aza-bicyclo[3.2.1]oct-2-ene hydrochloric acid salt**

A mixture of (±)-8-methyl-3-naphthalen-2-yl-8-aza-bicyclo[3.2.1]oct-2-ene (3.8 g, 15 mmol), toluene (50 ml) and 2,2,2-trichloroethylchloroformate (6.2 ml, 45 mmol) was stirred at reflux for 15 h. The mixture was allowed to cool to room temperature followed by addition of water (50 ml). The phases were separated and the organic phase was washed with water (2 x 50 ml). The organic phase was evaporated and was combined with acetic acid (20 ml), water and zinc (2.5 g, 38.2 mmol) and was stirred at room temperature for 3 days. The mixture was made alkaline by adding concentrated ammonia (100 ml) and extracted with diethylether (2 x 100 ml). The organic phase was dried and evaporated. The free base was converted to the hydrochloric acid salt. Yield 550 mg (16%). Mp >270°C.

**Method D**

**(+)-3-Naphthalen-2-yl-8-aza-bicyclo[3.2.1]oct-2-ene D-tartaric acid salt**

A mixture of (-)-8-methyl-3-(naphthalen-2-yl-)-8-aza-bicyclo[3.2.1]oct-2-ene (3.4 g, 13.6 mmol), 2,2,2-trichloroethylchloroformate (5.6 ml, 40.9 mmol) and toluene (50 ml) was stirred at room temperature for 1 h. The mixture was stirred at 100°C for 15 h. Water (100 ml) was added and the mixture was stirred for 20 min at room temperature. The organic phase was separated and was washed with water (2 x 50 ml). The organic phase was evaporated. Zinc-dust (4.4 g, 68.2 mmol), acetic acid (50 ml) and water (10 ml) was added followed by stirring for 15 h. The mixture was made alkaline by adding ice (100 g) and concentrated ammonia (100 ml). The mixture was extracted with dichloromethane (2 x 50 ml) and was isolated as an oil (yield 1.37 g, 5.8 mmol, 43%). Ethanol (50 ml, 96%) and D-tartaric acid (0.90 g, 6.0 mmol) was added and was heated to reflux where the mixture became clear and was allowed to crystallize and was isolated by filtration. $[\alpha]_D^{25}$ = (+)-33.1° (free base). Yield (calc.from the free base) 2.11 g (95%). Mp 183-189°C.

**(+)-3-(6-Methoxy-naphthalen-2-yl)-8-aza-bicyclo[3.2.1]oct-2-ene D-tartaric acid salt**

Was prepared according to method D. Mp Mp 213.9-216.3°C.

**(-)-3-Naphthalen-2-yl-8-aza-bicyclo[3.2.1]oct-2-ene L-tartaric acid salt**

Was prepared according to method D from (+)-8-methyl -3-(naphthalen-2-yl) -8-azabicyclo[3.2.1]oct-2-ene and L-tartaric acid Yield 41 %. $[\alpha]_D^{25}$ (free base). Mp (L-tartaric acid salt): 173.6-184.9°C.

**(-)-3-(6-Methoxy-naphthalen-2-yl)-8-aza-bicyclo[3.2.1]oct-2-ene D-tartaric acid salt**

Was prepared according to method D. Mp 213.4-214.4°C.

**Method E**

**(-)-8-Methyl-3-(naphthalen-2-yl)-8-aza-bicyclo[3.2.1]oct-2-ene hydrochloric acid salt**

A mixture of (-)-8-methyl-3-(trifluoromethylsulfonyloxy)-8-aza-bicyclo[3.2.1]oct-2-ene, (4.5 g 16.7 mmol), 1,2-dimethoxyetane (60 ml), 2-naphthylboronic acid (3.4 g, 20 mmol), potassium carbonate (8.5 g, 61.5 mmol), lithium chloride (0.85 g, 20.0 mmol) and water (30 ml) was bubbled through with argon for 10 min. Pd(PPh3)4 (0.05 g, 0.043mmol) was added followed by reflux for 1 h. The mixture was allowed to cool to room temperature. Water (100 ml) was added followed by extraction with diethyl ether (2 x 50 ml). The organic phase was washed with water (2 x 50 ml), dried and

evaporated. Yield 3.43 g (100%). The hydrochloric acid salt was precipitated by adding a mixture of HCl in ethanol. $[\alpha]_D^{25}$ = (–)-27.1° (free base). Yield 3.83 g (92%). Mp 265°C.

**(-)-3-(6-Methoxy-naphthalen-2-yl)-8-methyl-8-aza-bicyclo[3.2.1]oct-2-ene hydrochloric acid salt**

Was prepared according to method E. Mp 249.7°C.

**(+)-8-Methyl-3-(naphthalene-2-yl)-8-aza-bicyclo[3.2.1]oct-2-ene-hydrochloric acid salt**

Was prepared according to method E from (+)-8-methyl-3-(trifluoromethylsulfonyloxy)-8-aza-bicyclo[3.2.1]oct-2-ene. $[\alpha]_D^{25}$ = (+)-26.4° (free base).

**(+)-3-(6-Methoxy-naphthalen-2-yl)-8-methyl-8-aza-bicyclo[3.2.1]oct-2-ene hydrochloric acid salt**

Was prepared according to method E. Mp 249°C.

**Method F**

**(-)-8-Methyl-3-(trifluoromethylsulfonyloxy)-8-aza-bicyclo[3.2.1]oct-2-ene**

To a stirred mixture of [S-(R*, R*)](-)-bis-α-methyl-benzylamine hydrochloric acid salt ($[\alpha]_D^{25}$ = (–)-73.2°) (86.5 g, 0.33 mmol) and tetrahydrofuran (1500 ml) was added at <5°C : butyllithium (264 ml, 2.5 M) during 30 min. The mixture was stirred at 0°C for 1 h. The mixture was cooled to -70°C and tropinone (47.3 g, 0.34 mol) solved in tetrahydrofuran (200 ml) was added over a period of 90 min. The mixture was stirred for 3 h at -70°C. N-phenylbis-trifluoromethanesulfonimid (128.6 g, 0.36 mol) solved in tetrahydrofuran (300 ml) was added to the mixture <70°C over 1.5 h time period. The mixture was allowed to reach room temperature over night. Water (4 L) was added followed by extraction with diethylether (2 x 2 L). The organic phase was washed with water (2 x 1 L). The organic phase was dried and evaporated. The crude mixture (187 g) of the title product and the chiral amine was separated by silica gel (1 kg) column chromatography using ethyl acetate initially in order to eluate the chiral amine and then a mixture of methanol and dichloromethane (2 : 8) for the chiral triflate. The chiral triflate was isolated in 85% (0.29 mol). The chiral amine was re-isolated and precipitated as hydrochloric acid salt. Yield 53.9 g (55%). $[\alpha]_D^{25}$ = (–)-71.9°).

**(+)-8-Methyl-3-(trifluoromethylsulfonyloxy)-8-aza-bicyclo[3.2.1]oct-2-ene**

Was prepared according to method F using the other chiral amine [R-(R*, R*)](+)-bis-α-methyl-benzylamine hydrochloric acid salt ($[\alpha]_D^{25}$ = (+)-73.8°).

**(+)-6-(8-Aza-bicyclo[3.2.1]oct-2-en-3-yi)-naphthalen-2-ol hydrochloric acid salt**

A mixture of (-)-3-(6-methoxy-naphthalen-2-yl)-8-aza-bicyclo[3.2.1]oct-2-ene D-tartaric acid salt (1.1 g, 2.64 mmol), concentrated ammonia (0.5 ml) and water (50 ml) was extracted with dichloromethane, dried and evaporated. To a mixture of the free base of (-)-3-(6-methoxy-naphthalen-2-yl)-8-aza-bicyclo[3.2.1]oct-2-ene and dichloromethane (25 ml), was added: borontribromide (3 ml, 1 M in dichloromethane) at -15°C. The mixture was stirred at room temperature for 15 h. The mixture was made alkaline by adding aqueous ammonia and the free base was filtered as a solid. The corresponding hydrochloride was precipitated by adding ethanol (15 ml) and concentrated hydrochloric acid. Yield 0.49 g (64 %). LC-ESI-HRMS of [M+H]+ shows 252.1396 Da. Calc. 252.138839 Da, dev. 3 ppm.

**Test Example**

**In vitro inhibition activity**

A number of compounds were tested for their ability to inhibit the reuptake of the monoamine neurotransmitters dopamine(DA) noradrenaline(NA) and serotonine(5-HT) in synaptosomes as described in WO 97/16451.

The test values are given as $IC_{50}$ (the concentration ($\mu$M) of the test substance which inhibits the specific binding of $^3$H-DA, $^3$H-NA, or $^3$H-5-HT by 50%).

Test results obtained by testing selected compounds of the present invention appear from the below table:

**Table 1**

| Test compound | DA-uptake $IC_{50}(\mu M)$ | NA-uptake $IC_{50}(\mu M)$ | 5-HT-uptake $IC_{50}(\mu M)$ |
|---|---|---|---|
| 1st compound of method A; (+)-3-(4-Chlorophenyl)-8-H-8-aza-bicyclo[3.2.1]oct-2-ene | 0.26 | 0.028 | 0.010 |
| 1st compound of method D; (+)-3-Naphthalen-2-yl-8-aza-bicyclo[3.2.1]oct-2-ene | 0.058 | 0.013 | 0.00034 |
| 1st compound of method E; (-)-8-Methyl-3-(naphthalen-2-yl)-8-aza-bicyclo[3.2.1]oct-2-ene | 0.034 | 0.018 | 0.00023 |

**Claims**

1. An enantiopure compound of Formula I

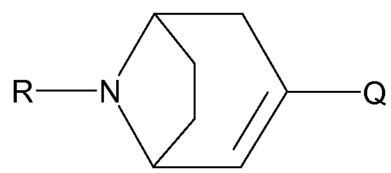

(I)

or a pharmaceutically acceptable salt thereof; wherein
R represents hydrogen or alkyl;
which alkyl is optionally substituted with one or more substituents independently selected from the group consisting of:

halo, trifluoromethyl, trifluoromethoxy, cyano, hydroxy, amino, nitro, alkoxy, cycloalkoxy, alkyl, cycloalkyl, cycloalkylalkyl, alkenyl and alkynyl; and

Q represents an aryl group;
which aryl group is optionally substituted with one or more substituents independently selected from the group consisting of:

halo, trifluoromethyl, trifluoromethoxy, cyano, nitro, hydroxy, alkoxy, cycloalkoxy, alkoxyalkyl, cycloalkoxyalkyl, alkyl, cycloalkyl, cycloalkylalkyl, alkenyl, alkynyl, -NR'R", -(C=O)NR'R" or - NR'(C=O)R";
wherein R' and R" independent of each other are hydrogen or alkyl.

2. The chemical compound of claim 1, wherein R represents hydrogen or alkyl.

3. The chemical compound of claims 1 or 2, wherein Q represents phenyl, which phenyl group is optionally substituted with one or more substituents independently selected from the group consisting of:

halo, trifluoromethyl, trifluoromethoxy, cyano, hydroxy and alkoxy.

**4.** The chemical compound of claims 1 or 2, wherein Q represents naphthyl, which naphthyl group is optionally substituted with one or more substituents independently selected from the group consisting of:

halo, trifluoromethyl, trifluoromethoxy, cyano, hydroxy and alkoxy.

**5.** The chemical compound of claims 1 or 2, wherein Q represents phenyl, which phenyl group is optionally substituted with one or two halo.

**6.** The chemical compound of claims 1 or 2, wherein Q represents naphthyl, which naphthyl group is optionally substituted with hydroxy or alkoxy.

**7.** The chemical compound of claim 1, being an enantiopure compound of 3-(4-Chlorophenyl)-8-H-8-aza-bicyclo[3.2.1]oct-2-ene; 3-Naphthalen-2-yl-8-aza-bicyclo[3.2.1]oct-2-ene; 3-(2-Chlorophenyl)-8-H-8-azabicyclo[3.2.1]oct-2-ene; 3-(2,3-Dichlorophenyl)-8-H-8-azabicyclo[3.2.1]oct-2-ene; 3-(3-Chlorophenyl)-8-H-8-azabicyclo[3.2.1]oct-2-ene; 3-(3,4-Dichlorophenyl) 8-H-8-azabicyclo[3.2.1]oct-2-ene; 3-(4-Chlorophenyl)-8-methyl-8-aza-bicyclo[3.2.1]oct-2-ene; 3-(2-Chlorophenyl)-8-methyl-8-azabicyclo[3.2.1]oct-2-ene; 3-(2,3-Dichlorophenyl)-8-methyl-8-azabicyclo[3.2.1]oct-2-ene; 3-(3,4-Dichlorophenyl)-8-methyl-8-azabicyclo[3.2.1]oct-2-ene; 3-(3-Chlorophenyl)-8-methyl-8-azabicyclo[3.2.1]oct-2-ene; 3-(6-Methoxy-naphthalen-2-yl)-8-aza-bicyclo[3.2.1]oct-2-ene; 8-Methyl-3-(naphthalen-2-yl)-8-aza-bicyclo[3.2.1]oct-2-ene; 3-(6-Methoxy-naphthalen-2-yl)-8-methyl-8-aza-bicyclo[3.2.1]oct-2-ene;
or a pharmaceutically acceptable salt thereof.

**8.** The chemical compound of claim 1, being
enantiopure (+)-3-(4-Chlorophenyl)-8-H-8-aza-bicyclo[3.2.1]oct-2-ene; enantiopure (-)-3-(4-Chlorophenyl)-8-H-8-aza-bicyclo[3.2.1]oct-2-ene; enantiopure (+)-3-(2-Chlorophenyl)-8-H-8-azabicyclo[3.2.1]oct-2-ene; enantiopure (+)-3-(2,3-Dichlorophenyl)-8-H-8-azabicyclo[3.2.1]oct-2-ene; enantiopure (+)-3-(3-Chlorophenyl)-8-H-8-azabicyclo[3.2.1]oct-2-ene; enantiopure (+)-3-(3,4-Dichlorophenyl) 8-H-8-azabicyclo[3.2.1]oct-2-ene; enantiopure (-)-3-(2-Chlorophenyl)-8-H-8-azabicyclo[3.2.1]oct-2-ene; enantiopure (-)-3-(2,3-Dichlorophenyl)-8-H-8-azabicyclo[3.2.1]oct-2-ene; enantiopure (-)-3-(3-Chlorophenyl)-8-H-8-azabicyclo[3.2.1]oct-2-ene; enantiopure (-)-3-(3,4-Dichlorophenyl)-8-H-8-azabicyclo[3.2.1]oct-2-ene; enantiopure (-)-3-(4-Chlorophenyl)-8-methyl-8-aza-bicyclo[3.2.1]oct-2-ene; enantiopure (-)-3-(2-Chlorophenyl)-8-methyl-8-azabicyclo[3.2.1]oct-2-ene; enantiopure (-)-3-(2,3-Dichlorophenyl)-8-methyl-8-azabicyclo[3.2.1]oct-2-ene; enantiopure (-)-3-(3,4-Dichlorophenyl)-8-methyl-8-azabicyclo[3.2.1]oct-2-ene; enantiopure (+)-3-(4-Chlorophenyl)-8-methyl-8-aza-bicyclo[3.2.1]oct-2-ene; enantiopure (+)-3-(2-Chlorophenyl)-8-methyl-8-azabicyclo[3.2.1]oct-2-ene; enantiopure (+)-3-(2,3-Dichlorophenyl)-8-methyl-8-azabicyclo[3.2.1]oct-2-ene; enantiopure (+)-3-(3-Chlorophenyl)-8-methyl-8-azabicyclo[3.2.1]oct-2-ene; enantiopure (+)-3-(3,4-Dichlorophenyl)-8-methyl-8-azabicyclo[3.2.1]oct-2-ene;
enantiopure (+)-3-Naphthalen-2-yl-8-aza-bicyclo[3.2.1]oct-2-ene;
enantiopure (+)-3-(6-Methoxy-naphthalen-2-yl)-8-aza-bicyclo[3.2.1]oct-2-ene; enantiopure (-)-3-Naphthalen-2-yl-8-aza-bicyclo[3.2.1]oct-2-ene;
enantiopure (-)-3-(6-Methoxy-naphthalen-2-yl)-8-aza-bicyclo[3.2.1]oct-2-ene; enantiopure (-)-8-Methyl-3-(naphthalen-2-yl)-8-aza-bicyclo[3.2.1]oct-2-ene; enantiopure (-)-3-(6-Methoxy-naphthalen-2-yl)-8-methyl-8-aza-bicyclo[3.2.1]oct-2-ene;
enantiopure (+)-8-Methyl-3-(naphthalen-2-yl)-8-aza-bicyclo[3.2.1]oct-2-ene; enantiopure (+)-3-(6-Methoxy-naphthalen-2-yl)-8-methyl-8-aza-bicyclo[3.2.1]oct-2-ene;
or a pharmaceutically acceptable salt thereof.

**9.** A pharmaceutical composition, comprising a therapeutically effective amount of a compound of any one of claims 1-8, or a pharmaceutically acceptable salt thereof, together with at least one pharmaceutically acceptable carrier, excipient or diluent.

**10.** Use of the chemical compound of any of claims 1-8, or a pharmaceutically acceptable salt thereof, for the manufacture of a medicament.

**11.** The use according to claim 10, for the manufacture of a pharmaceutical composition for the treatment, prevention or alleviation of a disease or a disorder or a condition which disease, disorder or condition is mood disorder, depression, atypical depression, depression secondary to pain, major depressive disorder, dysthymic disorder, bipolar disorder, bipolar I disorder, bipolar II disorder, cyclothymic disorder, mood disorder due to a general medical condition,

substance-induced mood disorder, pseudodementia, Ganser's syndrome, obsessive compulsive disorder, panic disorder, panic disorder without agoraphobia, panic disorder with agoraphobia, agoraphobia without history of panic disorder, panic attack, memory deficits, memory loss, attention deficit hyperactivity disorder, obesity, anxiety, generalized anxiety disorder, eating disorder, Parkinson's disease, parkinsonism, dementia, dementia of ageing, senile dementia, Alzheimer's disease, acquired immunodeficiency syndrome dementia complex, memory dysfunction in ageing, specific phobia, social phobia, social anxiety disorder, post-traumatic stress disorder, acute stress disorder, drug addiction, drug abuse, cocaine abuse, nicotine abuse, tobacco abuse, alcohol addiction, alcoholism, kleptomania, pain, chronic pain, inflammatory pain, neuropathic pain, migraine pain, tension-type headache, chronic tension-type headache, pain associated with depression, fibromyalgia, arthritis, osteoarthritis, rheumatoid arthritis, back pain, cancer pain, irritable bowel pain, irritable bowel syndrome, post-operative pain, post-mastectomy pain syndrome (PMPS), post-stroke pain, drug-induced neuropathy, diabetic neuropathy, sympathetically-maintained pain, trigeminal neuralgia, dental pain, myofacial pain, phantom-limb pain, bulimia, premenstrual syndrome, premenstrual dysphoric disorder, late luteal phase syndrome, post-traumatic syndrome, chronic fatigue syndrome, urinary incontinence, stress incontinence, urge incontinence, nocturnal incontinence, sexual dysfunction, premature ejaculation, erectile difficulty, erectile dysfunction, premature female orgasm, restless leg syndrome, periodic limb movement disorder, eating disorders, anorexia nervosa, sleep disorders, pervasive developmental disorders, autism, Asperger's disorder, Rett's disorder, childhood disintegrative disorder, learning disabilities, motor skills disorders, mutism, trichotillomania, narcolepsy, post-stroke depression, stroke-induced brain damage, stroke-induced neuronal damage, Gilles de la Tourettes disease, tinnitus, tic disorders, body dysmorphic disorders, oppositional defiant disorder or post-stroke disabilities.

Europäisches Patentamt
European Patent Office
Office européen des brevets

# EUROPEAN SEARCH REPORT

Application Number
EP 10 18 0734

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SMITH D F ET AL: "UPTAKE AND DISTRIBUTION OF A NEW SSRI, NS2381, STUDIED BY PET IN LIVING PORCINE BRAIN", EUROPEAN NEUROPSYCHOPHARMACOLOGY, ELSEVIER SCIENCE PUBLISHERS BV, AMSTERDAM, NL, vol. 9, no. 4, 1999, pages 351-359, XP009055589, ISSN: 0924-977X * page 353; table 1 * ----- | 1-11 | INV. C07D451/02 A61K31/46 A61P25/00 |
| X | SMITH D F ET AL: "Synthesis and in vivo evaluation (pig) of the selective serotonin reuptake inhibitor (11C)NS 2456", JOURNAL OF LABELLED COMPOUNDS AND RADIOPHARMACEUTICALS, vol. 44, no. Supplement 1, May 2001 (2001-05), pages S198-S200, XP008066561, & FOURTEENTH INTERNATIONAL SYMPOSIUM ON RADIOPHARMACEUTICAL CHEMISTRY; INTERLAKEN, SWITZERLAND; JUNE 10-15, 2001 ISSN: 0362-4803 * page S198, experimental * ----- | 1-11 | TECHNICAL FIELDS SEARCHED (IPC) C07D A61K A61P |
| X | WO 00/32600 A (NEUROSEARCH A/S; PETERS, DAN; OLSEN, GUNNAR, M; NIELSEN, SIMON, FELDBA) 8 June 2000 (2000-06-08) * claim 5 * ----- | 1-11 | |
| A,D | WO 97/13770 A (NEUROSEARCH A/S; MOLDT, PETER; SCHEEL-KRUEGER, JOERGEN; OLSEN, GUNNAR,) 17 April 1997 (1997-04-17) * page 12; table 2 * ----- | 1-11 | |

The present search report has been drawn up for all claims

1

| Place of search | Date of completion of the search | Examiner |
|---|---|---|
| The Hague | 19 November 2010 | Duval, Eric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 18 0734

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2010

| Patent document cited in search report | | Publication date | Patent family member(s) | | | Publication date |
|---|---|---|---|---|---|---|
| WO 0032600 | A | 08-06-2000 | AT | 259804 | T | 15-03-2004 |
| | | | AU | 761055 | B2 | 29-05-2003 |
| | | | AU | 1376100 | A | 19-06-2000 |
| | | | CA | 2342621 | A1 | 08-06-2000 |
| | | | CN | 1326456 | A | 12-12-2001 |
| | | | DE | 69914935 | D1 | 25-03-2004 |
| | | | DE | 69914935 | T2 | 01-10-2009 |
| | | | EP | 1133494 | A1 | 19-09-2001 |
| | | | JP | 2002531456 | T | 24-09-2002 |
| | | | NZ | 510287 | A | 30-05-2003 |
| | | | US | 2002035122 | A1 | 21-03-2002 |
| WO 9713770 | A | 17-04-1997 | AT | 362931 | T | 15-06-2007 |
| | | | AU | 709327 | B2 | 26-08-1999 |
| | | | AU | 7291796 | A | 30-04-1997 |
| | | | BR | 9610960 | A | 02-03-1999 |
| | | | CA | 2233541 | A1 | 17-04-1997 |
| | | | CN | 1199400 | A | 18-11-1998 |
| | | | CZ | 9800758 | A3 | 11-11-1998 |
| | | | DE | 69637097 | T2 | 20-09-2007 |
| | | | DK | 0859777 | T3 | 03-09-2007 |
| | | | EE | 9800062 | A | 17-08-1998 |
| | | | EP | 0859777 | A1 | 26-08-1998 |
| | | | HU | 9802433 | A2 | 28-04-1999 |
| | | | IL | 123583 | A | 31-07-2003 |
| | | | IS | 4681 | A | 04-03-1998 |
| | | | JP | 10512589 | T | 02-12-1998 |
| | | | JP | 3462505 | B2 | 05-11-2003 |
| | | | KR | 100274829 | B1 | 15-12-2000 |
| | | | NO | 980919 | A | 08-06-1998 |
| | | | NZ | 320216 | A | 28-05-1999 |
| | | | PL | 326195 | A1 | 31-08-1998 |
| | | | RU | 2157372 | C2 | 10-10-2000 |
| | | | SK | 28798 | A3 | 09-09-1998 |
| | | | TR | 9800628 | T2 | 21-07-1998 |
| | | | UA | 63894 | C2 | 16-02-2004 |
| | | | US | 6100275 | A | 08-08-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9713770 A
- WO 9730997 A
- WO 9716451 A

### Non-patent literature cited in the description

- Remington's Pharmaceutical Sciences. Maack Publishing Co,